(19) Europäisches Patentamt
European Patent Office
Office européen des brevets

(11) EP 0 938 894 A1

(12) EUROPEAN PATENT APPLICATION

(43) Date of publication:
01.09.1999 Bulletin 1999/35

(51) Int. Cl.$^6$: A61K 9/00

(21) Application number: 98200482.2

(22) Date of filing: 16.02.1998

(84) Designated Contracting States:
AT BE CH DE DK ES FI FR GB GR IE IT LI LU MC NL PT SE
Designated Extension States:
AL LT LV RO SI

(71) Applicant: Biomat B.V.
6229 ER Maastricht (NL)

(72) Inventor: Koole, Leo H.
6271 EC Gulpen (NL)

(74) Representative:
Smulders, Theodorus A.H.J., Ir. et al
Vereenigde Octrooibureaux
Nieuwe Parklaan 97
2587 BN 's-Gravenhage (NL)

(54) Implantable radiopaque device and use thereof

(57)     The invention concerns an implantable polymeric device for the controlled local delivery of drugs, having the following features:

the device comprises a hollow sphere, having a cavity and a polymeric wall
its wall is rubbery, porous, and self healing
its wall is biocompatible
its wall is constructed from a polymeric material with radio-opacity.

**Description**

The invention is directed to an implantable device for controlled local drug delivery and to the use of such a device in controlled local drug delivery.

Controlled local drug delivery is seen as a promising method for administering drugs at a specific spot in the body, for targeting drugs and for continuous delivery of drugs over a longer period of time.

Numerous strategies to achieve controlled local drug delivery have been disclosed.

US patent 5709874 (SR Hanson, NA Scott, SB King, III, LA Harker - Atlanta, GA) discloses a device for the local delivery of a substance into a natural tissue conduit in the mammalian body. The device has a substance delivery segment which provides a means for locally delivering a substance into the boundary layer of fluid flowing through the conduit without disrupting the fluid flow therethrough. For example, an indwelling catheter is provided for endovascular delivery of a substance locally to a targeted treatment area. Also provided are methods of locally delivering a substance into a natural tissue conduit in the mammalian body utilizing the device of the present invention. Noteworthy are also the references cited in this patent text.

US Patent 5702717 (Y Cha, YK Choi, YH Bae - Salt Lake city, UT) describes a system and method for the parenteral delivery of a drug in a biodegradable polymeric matrix to a warm blooded animal as a liquid with the resultant formation of a gel depot for the controlled release of the drug. The system comprises an injectable biodegradable block copolymeric drug delivery liquid having reverse thermal gelation properties. The delivery liquid is an aqueous solution having dissolved or dispersed therein an effective amount of a drug intimately contained in a biodegradable block copolymer matrix. The copolymer has a reverse gelation temperature below the body temperature of the animal to which it is administered and is made up of (i) a hydrophobic A polymer block comprising a member selected from the group consisting of poly(.alpha.-hydroxy acids) and poly(ethylene carbonates) and (ii) a hydrophilic B polymer block comprising a polyethylene glycol. Prior to use the liquid is maintained at a temperature below the reverse gelation temperature of the block copolymer. The liquid is parenterally administered into the animal by intramuscular, intraperitoneal, subcutaneous or similar injection with the liquid forming a gel depot of the drug and biodegradable block polymer as the temperature of the liquid is raised by the body temperature of the animal the reverse gelation temperature of the block copolymer. The drug is released at a controlled rate from the copolymer which biodegrades into non-toxic products. The degradation rate can be adjusted by proper selection of the poly($\alpha$-hydroxy acid) utilized in forming the biodegradable hydrophilic A block. Noteworthy are also the references cited in this patent text.

US Patent 5656296 (SU Khan, P Ying, RU Nesbitt, MB Fawzi, J Weiss - Morris Plains, NJ) describes an invention pertaining to a dual control sustained release drug delivery system which comprises a core and a porous coating layer over the core, wherein the coated core comprises a core comprising in percentages by weight of the core composition (a) a medicament present in an amount from about 60% to about 90%; (b) an edible material having a melting point from about 25°C to about 100°C selected from the group consisting of (i) fatty acids having an iodine value from about 1 to about 10, (ii) natural waxes, (iii) synthetic waxes, and (iv) mixtures thereof, present in an amount from about 5% to about 40%; and (B) a porous coating layer over the core comprising in percentages by weight of the coating layer composition (a) a pH-independent water-insoluble polymer present in an amount from about 40% to about 80%; and (b) a water-soluble film forming polymer present in an amount from about 20% to about 60%. Noteworthy are also the references cited in this patent text.

US Patent 5607418 (RC Arzbaecher - Chicago IL) discloses an implantable drug delivery apparatus including a housing that has a housing chamber. An outer deformable body having a reserve chamber is mounted within the housing chamber. An inner deformable body having a dispensing chamber is mounted within the reserve chamber. A dispensing valve is actuated to an open position to allow a fluidic drug to flow from the dispensing chamber, through the dispensing valve and through a catheter into a body of a patient. The fluidic drug is discharged from the dispensing chamber at a dispensing mass flow rate which is greater than a refilling mass flowrate of the fluidic drug passing from the reserve chamber to the dispensing chamber, when the dispensing valve is in an open position. When the dispensing valve is in a closed position, the fluidic drug is prevented from discharging from the dispensing chamber while a recharging amount of the fluidic drug is capable of flowing from the reserve chamber to the dispensing chamber, until the dispensing chamber is at least partially filled. The outer deformable body is normally forced or urged in a direction or manner that tends to reduce a volume of the reserve chamber. Noteworthy are also the references cited in this patent text.

Most of the prior art references suffer from the drawback that the devices can only be used once. One group of devices break down in the body during the delivery of the drug. Another group of devices remains intact and has to be replaced operatively after use.

It is an aim of the invention to provide an implantable device for local controlled delivery of drugs that does not suffer from these drawbacks.

It is a further aim of the invention to provide such a device that can easily be recharged after the drug has been delivered, without complicated operative procedures.

The invention provides an implantable device for local controlled drug delivery, comprising a hollow sphere having a polymeric wall, said wall being microporous, rubbery, self-healing, bio-compatible and radiopaque.

Surprisingly it has been found that the device of the invention can be implanted in the body of a mammal and, after delivery of the drug (active ingredient) contained therein, can be repeatedly recharged in situ without removing the device from the body.

The unique feature of this device is that it can repeatedly be recharged in situ and can therefore be used for sustained local drug delivery, even if relatively high concentration of the drug are required.

Fields of application are, but are not limited to: (i) controlled local drug delivery via diffusion of drug molecules across the polymeric micoporous wall of the device, e.g. release of a cytostatic agent; (ii) encapsulation of cell cultures, e.g. Langerhans island cells, for the in situ production of insulin, as a novel concept for the artificial pancreas.

In its simplest form, the device is a polymeric hollow sphere with an outer diameter in the range of 1-40, preferably 5 - 20 mm with wall thickness in the range of 0.1 to 2.5, preferably 0.4 - 1.5 mm, but smaller and larger dimensions are also possible. The wall structure of the device is engineered on the basis of four essential considerations:

- the material is microporous, thus permitting drug diffusion across the wall;
- the material is rubbery and self-healing, i.e. the wall can be punched with a thin syringe needle, and subsequent withdrawal of the needle is followed by spontaneous closure of the punch-hole (much like in a septum); generally this means a glass transition temperature below body temperature, preferably below 0°C;
- the material is biocompatible and durable;
- the material is radio-opaque, i.e. capable of absorbing X-rays to such an extent that accurate localization of an implanted sphere is possible using X-ray fluoroscopic techniques routinely available in a modern hospital.

The key idea is that the physician can introduce two thin syringe needles into the device cavity in situ. A concentrated solution of the drug can then be injected through one of the needles, while the other serves as outlet to prevent build-up of pressure. When charging is complete and both needles are withdrawn, the diffusion process is essentially repeated.

Suitable polymers in the preparation of the hollow spheres are based on a class of radiopaque biomedical polymeric materials, preferably having a number average molecular weight of at least 7500. These radiopaque materials are either: (i) polymers of a monomer molecule that contains covalently-bound iodine, or (ii) copolymers in which at least one of the different monomers contains covalently-bound iodine, or (iii) terpolymers or polymers of even higher complexity, in which at least one of the different monomers contains covalently-linked iodine. More preferred polymers are defined in the dependent claims. In order to provide sufficient radio-opacity it is preferred that the polymer composition of the wall contains at least 5 wt.% of iodine.

Examples of these polymers are i.a. those used in the following examples. More in particular acrylate based polymers may be used, especially those containing covalently bound iodine, such as those disclosed in WO-A 9605872, the disclosure of which is incorporated herein by way of reference. More in particular the polymers used may be cross-linked.

The hollow spheres can be prepared in a conventional manner, or in the manner disclosed in the examples.

The invention also provides for a method for the controlled local delivery of an active ingredient, such as defined in the claims.

The invention is now elucidated on the basis of the figures and examples.

## Legends to the figures

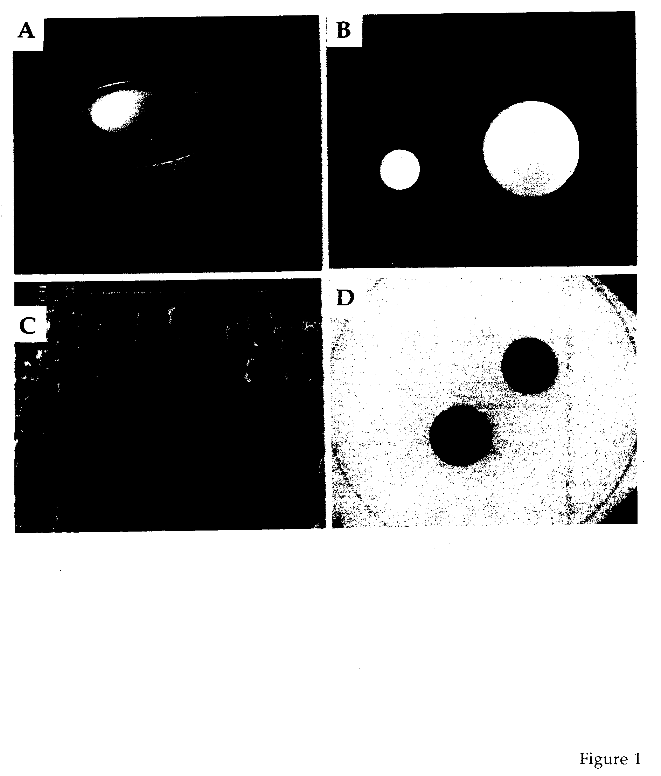

Fig. 1 A, One of the mould halves used for the preparation of the large hollow spheres. B, Photograph of the small and large hollow spheres. C, Scanning electron micrograph of the high-porosity wall material. Scale bar, 5 $\mu$m. D, X-ray fluoroscopic images of two large hollow spheres, placed on a glass Petri dish.

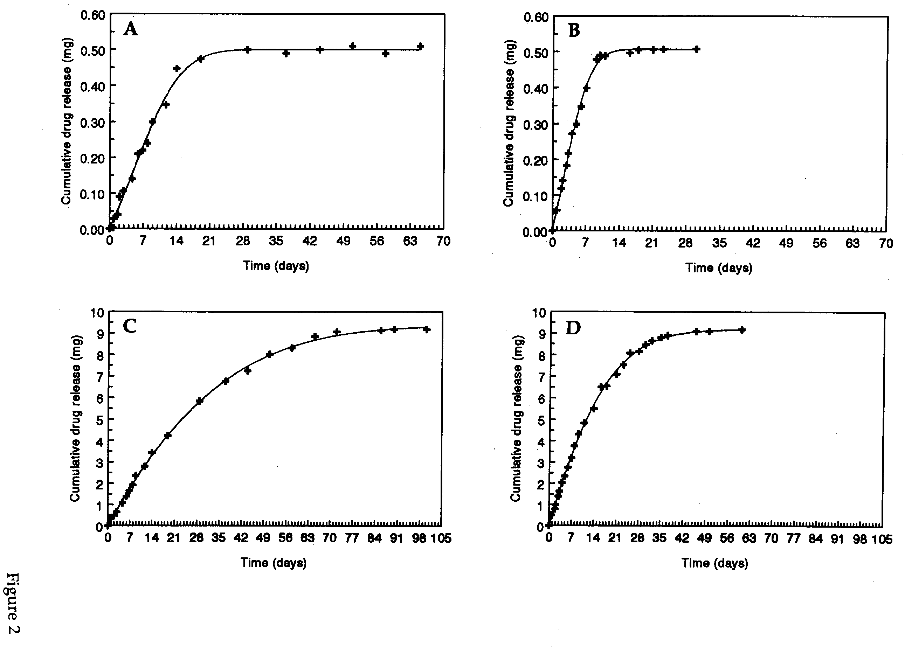

Fig. 2 Cumulative release profiles of 5-FU from the four different types of spheres. A, small spheres, low porosity. B, small spheres, high porosity. C, large spheres, low porosity. D, large spheres, high porosity.

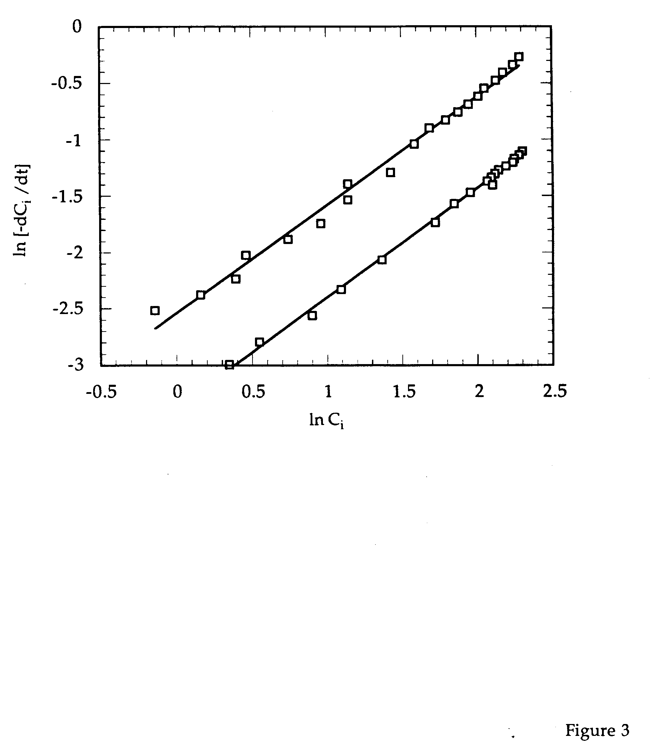

Fig.3 Plots of ln [-d$C_i$/dt] vs. ln $C_i$ derived from the experimental data on the release of 5-FU from the large reservoirs (high porosity and low porosity).

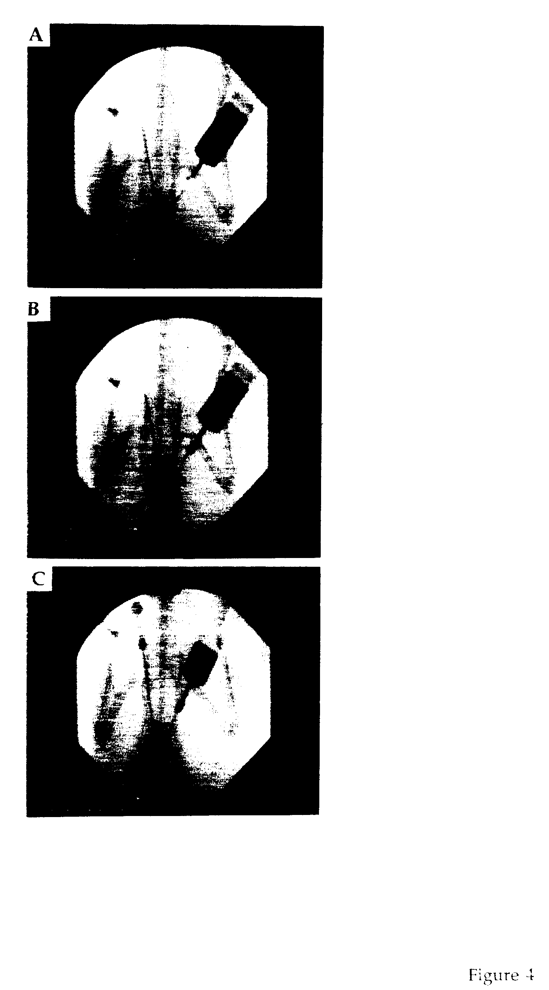

Fig. 4 X-ray fluoroscopic images recorded during charging of the reservoir in the peritoneum of rat # 7. A, Introduction of the two needle tips into the centre of the radio-opaque shell. This could be readily accomplished using real-time fluoroscopy. A slight deformation of the sphere could be detected twice, i.e. each time when the syringe needle penetrated the wall. B, Situation shortly after the start of the injection. At this stage, the reservoir contains some contrast agent, but the concentration is still relatively low. C, End of the charging procedure. The reservoir is now filled with contrast, and it is also seen that some of the contrast agent has left the reservoir via the second needle. After retraction of both needles, the reservoir re-assumed its perfect spherical shape, and also retained its original position.

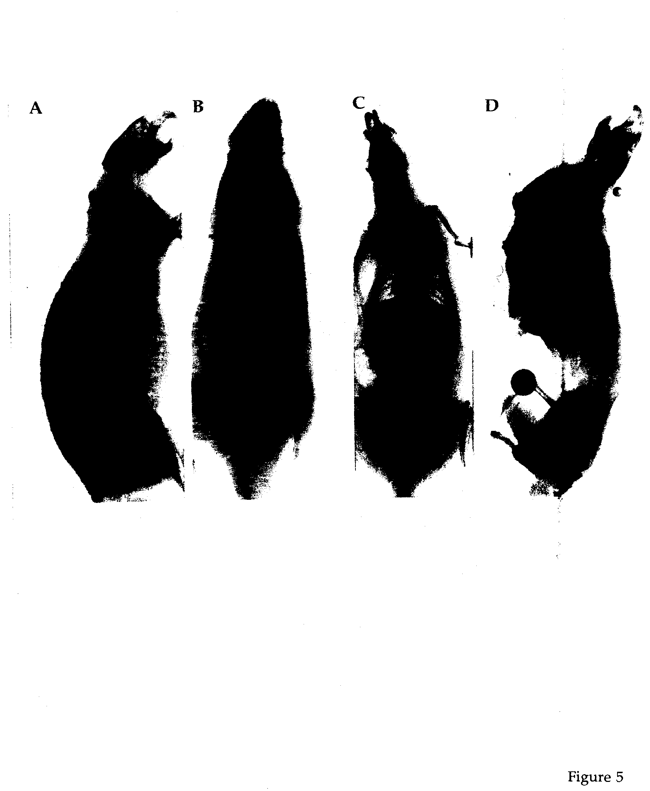

**Fig 5** X-ray fluoroscopic images of rat #7. **A**, Situation immediately prior to charging of both reservoirs with iopamidol, i.e. 4 weeks post-implantation. **B**, Situation 24 h after charging with iopamidol. **C**, Situation 26 weeks after charging. **D**, Situation 52 weeks after charging.

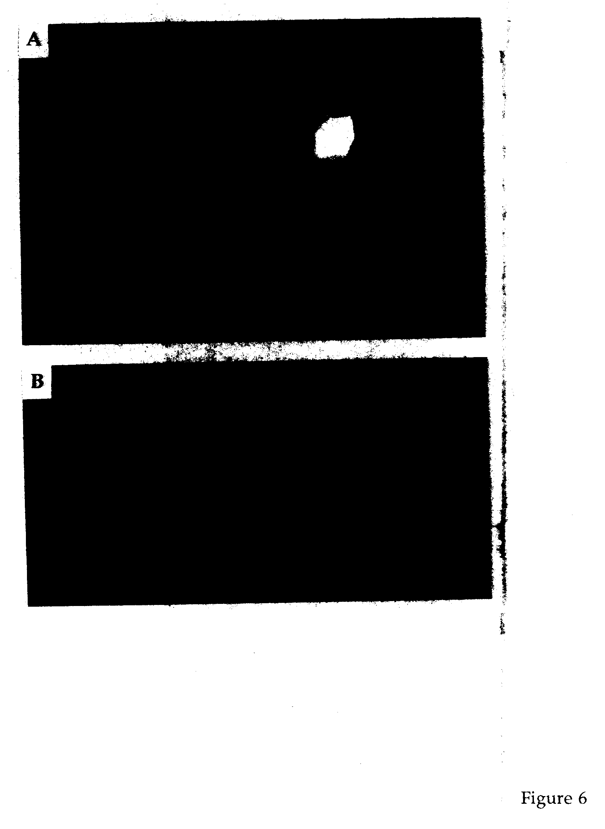

**Fig. 6** Scintigraphic images of the rabbit model. **A**, Lateral view recorded 2 h post implantation, clearly showing that the radioactivity is mainly concentrated inside the cavity of the implanted sphere. **B**, Anterior view 27 h post implantation (i.e. more than 2 times the radioactive half-life). At this stage, three hot spots are clearly discernable, i.e. the thyroid gland (two lobes), the sphere, and the bladder. Most of the iodide has left the delivery device at this stage (viz. Table 1), while the release process is still ongoing. Note that the feet of the animal are also slightly visible, due to contact with its own radioactive urine.

**EXAMPLE 1**.

Spheres were prepared in a mold (viz. Fig. 1A), through a radical copolymerization reaction of two reactive monomers, i.e. 2-hydroxyethyl methacrylate (HEMA), and 2-[2'-iodobenzoyloxy]-ethyl methacrylate (2-IEMA), in the molar ratio 4 : 1, respectively. Moreover, a relatively small amount (0.5 % by weight) of a bifunctional monomer, tetraethyleneglycol dimethacrylate, was added to the reaction mixture. Several considerations led us to choose this formulation, e.g. (1) three-dimensional networks composed of HEMA usually exhibit excellent biocompatibility and stability, (2) incorporation of > 20 mole % of 2-IEMA renders the resulting copolymer markedly radio-opaque, (3) copolymerization of HEMA and 2-IEMA proceeds readily and with high conversion, affording random Bernoullian-type copolymers. Note that each sphere wall is essentially a continuous three-dimensional molecular network; no glue is used in this method to prepare hollow spheres.

The reactive mixture used for the preparation of the spheres consists of: HEMA (5.91 g, 45.41 mmol), 2-IEMA (4.09 g, 11.36 mmol), tetraethyleneglycol dimethacrylate (0.050 g), $\alpha,\alpha'$-azobis(isobutyronitrile) (0.02841 mmol) and dimethylformamide (3.33 g for the low-porosity material, and 5.00 g for the high-porosity material). Thus, total monomer weight : solvent weight is 3 : 1 for the low-porosity material, and 2 : 1 for the high-porosity material. The small and large moulds were charged with 75 $\mu$L and 750 $\mu$L of the reactive mixture, respectively. The outer part of the moulds consisted of stainless steel. The inner part was made of poly(tetrafluoroethylene), a non-adhesive plastic that can be machined with excellent precision and reproducibility. Machining was performed on a computer-controlled lathe-mill. The closed moulds were randomly twirled for app. 90 min, while the temperature was increased from ambient to 100°C. Slow cooling and careful opening of the mould then afforded a transparent hollow sphere which was immediately immersed in a beaker containing app. 100 mL of doubly distilled water. The spheres were seen to immediately turn white-opaque upon contacting water. Each time, the cavity was immediately filled with water by means of a thin-needle (0.4 mm) syringe. The water in the beaker was magnetically stirred for 2 weeks, and the water was exchanged twice a day. Elemental analysis then revealed the absence of any trace of nitrogen, showing that dimethyl formamide was completely washed out. Prior to implantation, all reservoirs were filled with a sterile phosphate-buffered saline (PBS) solution, immersed in anhydrous ethanol for app. 300 s, and stored in sterile PBS at 37°C.

**EXAMPLE 2**.

Four different types of spheres were prepared and investigated. First, we distinguish two different sizes (small and large; Fig. 1B). The small spheres have the dimensions: outer diameter 5.5 mm, wall thickness 0.45 mm, inner volume 0.051 mL. Large spheres: outer diameter 14.0 mm, wall thickness 1.0 mm, inner volume 0.905 mL. It could be verified that the spheres had uniform wall thickness. Secondly, we distinguish two different wall porosities. Fig. 1C shows the structure of the highly porous wall, as determined with scanning electron microscopy. The pores are evenly distributed over the entire sample; their average diameter is 0.2 $\mu$m. The low-porosity wall structure is different in the sense that less pores are present; the pore size was found to be the same. The X-ray visibility of the spheres was checked with a Siemens Cardioscope U instrument. The spheres were placed on a Petri dish, which was put on top of a 15-cm thick layer of Plexiglass®. This polymer block absorbs X rays to approximately the same extent as the chest of an adult. Images like Fig. 1D were obtained, leaving no doubt that these spheres would be clearly detectable once implanted in the human body.

**EXAMPLE 3**.

The four types of spheres were charged with a concentrated solution of 5-fluorouracil (5-FU, 10 mg per mL), a clinically used antineoplastic drug. 5-FU is used *inter alia* for treatment of colorectal cancers. *In vitro* drug release monitoring was performed at 37°C, under exclusion of light, and under constant gentle roller mixing. Volumes of the release media were kept constant at 25 mL for the small spheres, and at 125 mL for the large ones. The concentration of 5-FU in the release medium (deionized water) was monitored spectrophotometrically at 267 nm ($\varepsilon$ = 6951 L/mol.cm). The 5-FU concentration in the release media was far below 10 % of its solubility, i.e. sink conditions were maintained throughout the experiments. Fig. 2 shows the resulting drug release profiles. Reproducibility was verified in several independent experiments (*vide infra*). Drug release from both large spheres was analyzed in more detail. To determine the order of release in these cases, we constructed plots of ln[-dCi /dt] versus ln $C_i$ (Figure 3); the order is then found as the slope of the resulting straight line. The best-fit equations of these lines are: y = -3.368 + 0.967 x (lower line, low porosity, according to Fig. 2C) , and y = -2.558 + 0.963 x (upper line, high porosity, according to Fig. 2D) , showing that the release is first order in both cases.

The self-healing properties of both wall structures were tested as follows: a 0.4-mm syringe needle was punctured and withdrawn at 10 different sites on the sphere's surface. Then, 2 punctured and 2 control (untreated) spheres were charged with 5-FU, and release of the drug was monitored in the course of time. It could be established that there was no accelerated drug release for the punctured spheres, as compared to the controls.

## EXAMPLE 4.

A series of animal experiments in order to investigate the biocompatibility and biostability of the material was performed. A further aim of these experiments was to find out whether the idea of recharging the drug reservoir *in situ* would be feasible, at least in the animal model. Both small and large spheres were implanted in rats; all implants were made of the radio-opaque materials with the lowest porosity. Seven male Wistar Kyoto rats of app. 300 g body weight were used. In rats #1 - 4, a small sphere was implanted subcutaneously in the neck. Rats #5 and 6 had a large sphere implanted in the peritoneum. Rat #7 received two spheres: a small one in the subcutus of the neck, and a large one intraperitoneally. All rats recovered quickly and behaved perfectly normal after the implantation. Rats #1 - 6 were sacrificed after 9 weeks. Microscopic evaluation of the explants and the surrounding tissues showed that the spheres were well tolerated by the subcutaneous or intraperitoneal tissues. No signs of any structural degradation were seen for any of the explants of rats #1 - 6. These results were completely in line with our previous data on the *in vivo* biocompatibility of radio-opaque polymeric networks based on 2-IEMA and HEMA.14

## EXAMPLE 5.

Rat #7 was used for a test of the assumed *in situ* rechargeability. The test was performed 4 weeks post implantation. The X-ray fluoroscopic images in Fig. 4 were recorded consecutively during filling of the radio-opaque reservoir in the peritoneum or rat #7 with a concentrated solution of iopamidol (Iopamiro® 370), a well-known iodine containing non-toxic contrast agent. The concentration of the contrast agent in the syringe was 755.2 mg / mL. The same procedure was carried out with the small sphere implanted subcutaneously in the neck. Charging proceeded smoothly in both cases (Fig. 4). Examination of the animal after 24 h showed that both reservoirs were still filled with contrast agent (Fig. 5B). There were no indications of any leakage, showing that the puncture holes close also *in situ.* After 26 weeks, the small reservoir showed a darker circumference in comparison with its interior, indicating that most of the contrast agent has been released. The large reservoir still appears as a completely black field (Fig 5C). After 52 weeks, both reservoir are essentially free of contrast agent (Fig. 5D). By accident, part of the knee joint is seen superimposed on the image of the large sphere. This provides additional evidence for the "emptiness" of the reservoir. The following points are also noteworthy: (1) Iopamidol is a larger molecule than 5-FU (the molar masses are 770 and 130g/mole, respectively). This explains why release of contrast is slower than release of 5-FU (compare Figs. 2B and D); (2) the reservoirs remained in their original position during the full experimental period. We anticipated that some migration, particularly of the sphere in the peritoneum, would have been possible; (3) The X-ray opacity of the reservoirs remained essentially unchanged during the full experimental period. This provides further evidence for the structural stability and integrity of the radio-opaque porous polymeric material.

## EXAMPLE 6.

A small sphere with low porosity was charged with aqueous Na[123]I (15 mCi), and implanted subcutaneously in the neck of an adult male rabbit (weight: 3.2 kg.). The rabbit was anaesthetized with an intramuscular dose of a mixture of 0.5 mL/kg ketamine hydrochloride. $^{123}$I is a $\gamma$-emitter with half-life of 13h, and *in vitro* experiments had shown that diffusion across the reservoir wall is much faster for [123-]I than for 5-FU or iopamidol, presumably since [123-]I is a smaller particle with a better solubility in water. A series of scintigraphic images was recorded, starting immediately after wound closure. Initially, merely a bright spot of radioactivity at the site of sphere was seen. After 2h, a small part of the radioactivity was spread over the entire body of the animal; only the bladder could be distinguished at this stage (Fig. 6A). Scintigraphy after 4h clearly showed three hot spots: the sphere, the bladder, and the thyroid gland. Later observations (at 22h, 27h, and 50h) showed essentially the same pattern (Fig. 6B), although the relative intensities of the hot spots changed considerably with time (Table 1). Our interpretation of these observations is that $^{123}$I ions are first released into tissues surrounding the charged sphere. Normal clearance, mainly toward the thyroid gland and the bladder, occurs thereafter. By analogy, we assume that this procedure can also be followed to locally administer a drug, e.g. a cytostatic agent. Of course, the availability of the drug, i.e. its concentration in the region near the release depot, will depend on both the release kinetics and the clearance. Whole-body scintigraphic images of the rabbit model were recorded on a single-head Siemens gamma camera equipped with a low-energy high resolution collimator. The energy window (15 %) for $^{123}$I was set at 158 keV. Images were recorded during app. 20 min, using a 1024 x 1024 data matrix. Data were stored on an optical/magnetic disc.

## EXAMPLE 7.

The rechargeable drug reservoir introduced here offers an alternative to at least three existing strategies to combat -for instance- solid brain tumors: temporary local release of a chemotherapeutic drug from of a biodegradable polymeric matrix, direct intratumoral injection of a drug, or local drug delivery via an Ommaya reservoir. One essential limitation of the new concept is, of course, that it must be possible to place one or more radio-opaque reservoirs within the tumor resection cavity, or close to a tumor which can not be surgically removed. However, implantation of a radio-opaque reservoir is likely to be impossible for deep-lying cystic lesions. Another serious point is that the radio-opaque plastic does not degrade in vivo, i.e. each depot must be regarded as a permanent implant. But the most important argument in favor of the new device is that its rechargeability makes prolonged local delivery of relatively large amounts of a drug possible. Furthermore, dedicated treatment strategies can be made feasible. For example, if the physician is able to assess the growth of a (residual) tumor, she or he can respond by adapting the daily amount of drug that is locally released. Changing a therapy is also conceivable, i.e., the drug reservoir could be emptied and recharged with a different cytostatic agent. We realize that the radio-opaque hollow spheres share these potential advantages with the well-known Ommaya reservoir, which already finds clinical use for local administration of drug tot cystic brain lesions and/or their aspiration. Some specific drawbacks of the Ommaya device (e.g., the high profile of its button that raises up the skin and may lead to skin necrosis or infection) do not occur with the radio-opaque hollow device disclosed herein.

## EXAMPLE 8. The radio-opaque device as described above can also

be used for the encapsulation and immunoisolation of relatively large colonies of cells, for instance pancreatic islets. Transport of nutrients occurs via diffusion across the porous wall of the device. The efflux of products of cellular metabolism (e.g. insulin) also proceeds through diffusion across the device wall, but in opposite direction. If the viability and function of encapsulated cells begins to cease, e.g. at the end of their life span, the contents of the device can be removed, using two syringes (*vide supra*), and a new batch of cells can be used to recharge/revitalise the system. Evidently, this recharging is also possible when the device is implanted: the device is radio-opaque and can easily and accurately be localised. Application of the new device eliminates several common problems which are normally encountered during transplantation of xenogenic cells (immunoattack and limited life span). Accurate control over the porosity (size and distribution of the pores) is essential.

Table 1

| Distribution of radioactivity in the rabbit measured as a function of time post implantation. | |
|---|---|
| Time post implantation (min) | counts thyroid gland / counts reservoir |
| 0 | 0 |
| 250 | 0.18 |
| 1296 | 1.37 |
| 1646 | 1.41 |
| 2987 | 2.56 |

**Claims**

1. An implantable polymeric device for the controlled local delivery of drugs, having the following features:

   • the device comprises a hollow sphere, having a cavity and a polymeric wall
   • its wall is rubbery, porous, and self healing
   • its wall is biocompatible
   • its wall is constructed from a polymeric material with radio-opacity.

2. A polymeric device for controlled drug delivery according to claim 1 wherein the wall structure comprises a copolymer or a terpolymer in which at least one radio-opaque molecular building block is present in the form of an acrylate structure having a side chain to which at least one iodine atome is covalently attached.

3. A polymeric device for controlled drug delivery according to claim 1 or 2 wherein the wall structure comprises a copolymer or a terpolymer in which a radio-opaque molecular building block is present in the form of a methacrylate structure having a side chain to which at least one iodine atome is covalently attached.

4. A polymeric device for controlled drug delivery according to claims 1-3 wherein the wall structure comprises a copolymer or a terpolymer in which a radio-opaque molecular building block is present in the form of a cyanoacrylate structure having a side chain to which at least one iodine atom is covalently attached.

5. A polymeric device for controlled drug delivery according to claims 1-4 wherein the wall structure comprises an insoluble three-dimensional polymeric network in which at least one radio-opaque molecular building block is present in the form of an acrylate structure having a side chain to which at least one iodine atom is covalently attached.

6. A polymeric device for controlled drug delivery according to claim 1 wherein the wall structure is an insoluble three-dimensional polymeric network in which at least one radio-opaque molecular building block is present in the form of a methacrylate structure having a side chain to which at least one iodine atom is covalently attached.

7. A polymeric device for controlled drug delivery according to claim 1 wherein the wall structure is an insoluble three-dimensional polymeric network in which the radio-opaque molecular building block is a cyanomethacrylate structure having a side chain to which at least one iodine atome is covalently attached.

8. A device according to any one of the claims 1-7, wherein the cavity is at least partly filled with an active ingredient.

9. A device according to claim 8, wherein the active ingredient is selected from the group of cytostatic agents.

10. A device according to any one of the claims 1-7, wherein the cavity is at least partly filled with cells.

11. A device according to claim 10, wherein the cavity contains an insulin-releasing system.

12. A method for the controlled local delivery of a drug, said process comprising implanting the device of claim 8 in the body of a mammal.

13. A method according to claim 12, said method additionally comprising the steps of determining the position of the device in the body after the device was at least partly discharged, and injecting a charge of active ingredient through the wall of the device, without removing the device from the mammalian body.

Figure 1

Figure 2

Figure 3

Figure 4

Figure 5

Figure 6

<table>
<tr><td colspan="2"><strong>European Patent Office</strong></td><td><strong>EUROPEAN SEARCH REPORT</strong></td><td><strong>Application Number</strong><br>EP 98 20 0482</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | WO 93 21902 A (SOMATIX THERAPY) 11 November 1993 * claim 86 * * page 23, paragraph 1 * * figure 16 * --- | 1-13 | A61K9/00 |
| A | D. HORÁK, ET AL.: "Hydrogels in endovascular embolization. III. Radiopaque spherical particles, their preparation and properties" BIOMATERIALS, vol. 8, no. 2, March 1987, pages 142-145, XP002076735 Guildford, GB * the whole document * --- | 1-13 | |
| A | D. HORÁK, ET AL.: "New radiopaque polyHEMA-based hydrogel particles" J. BIOMED. MATER. RES., vol. 34, no. 2, 1997, pages 183-188, XP002076736 * the whole document * --- | 1-13 | |
| A | A. JAYAKRISHNAN, ET AL.: "Preparation and evaluation or radiopaque hydrogel microspheres based on PHEMA/iothalamic acid and PHEMA/iopanoic acid as particulate emboli" J. BIOMED. MATER. RES., vol. 24, no. 8, 1990, pages 993-1004, XP002076737 * the whole document * --- -/-- | 1-13 | TECHNICAL FIELDS SEARCHED (Int.Cl.6) A61K |

The present search report has been drawn up for all claims

| Place of search | Date of completion of the search | Examiner |
|---|---|---|
| THE HAGUE | 8 September 1998 | Ventura Amat, A |

**European Patent Office**

## EUROPEAN SEARCH REPORT

Application Number

EP 98 20 0482

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | M. A. B. KRUFT, ET AL.: "Studies on two new radiopaque polymeric biomaterials" J. BIOMED. MATER. RES., vol. 28, no. 11, 1994, pages 1259-1266, XP002076738 * the whole document * | 1-13 | |
| | | | **TECHNICAL FIELDS SEARCHED (Int.Cl.6)** |

The present search report has been drawn up for all claims

| Place of search | Date of completion of the search | Examiner |
|---|---|---|
| THE HAGUE | 8 September 1998 | Ventura Amat, A |

EPO FORM 1503 03.82 (P04C01)